# EUROPEAN PATENT SPECIFICATION

(11) **EP 0 982 399 B1**
(45) Date of publication and mention of the grant of the patent: **26.10.2005**
(21) Application number: 98202876.3
(22) Date of filing: 27.08.1998
(51) Int. Cl.: C12N 15/12, C07K 14/47, C12N 5/10, C07K 16/18, G01N 33/50, G01N 33/563, A61K 48/00, A61K 38/17

(54) **Thyroid hormone transporters, agonists and antagonists thereof**
Transporterproteine spezifisch für Schilddrüsenhormone und deren Agonisten und Antagonisten
Protéines de transport d' hormone thyroidienne et leurs agonistes et antagonistes

(43) Date of publication of application: 01.03.2000
(73) Proprietor: Stichting tot bevordering van de wetenschap der Endocrinologie, 3015 GD Rotterdam (NL)
(72) Inventor: Krenning, Eric Paul, 3062 JC Rotterdam (NL); Hennemann, George, 3062 JP Rotterdam (NL); Docter, Roelof, 2904 SN Capelle aan den IJssel (NL); Visser, Theofilus Johannes, 3192 MB Hoogvliet (NL)
(74) Representative: Smulders, Theodorus A.H.J.

(56) References cited:
- WO-A-95/17905
- DOCTER,R., ET AL. : "expression of rat liver cell membrane transporters for thyroid hormone in Xenopus leavis oocytes" ENDOCRINOLOGY, vol. 138, no. 5, 1997, pages 1841-1846, XP002092885
- HENNEMANN, G., ET AL .: "the significance of plasma membrane transport in the bioavailability of thyroid hormone" CLINICAL ENDOCRINOLOGY, vol. 48, January 1998, pages 1-8, XP002092886
- MOL, J.A., ET AL. : "inhibition of iodothyronine transport into at liver cells by a monoclonal antibody" THE JOURNAL OF BIOLOGICAL CHEMISTRY, vol. 261, no. 17, 15 June 1986, pages 7640-7643, XP002092887
- FRIESEMA, E.C.H., ET AL. : "effects of organic anions (bile acids) on T3sulfamate (T3NS) and T4sulfamate (T4NS) uptake into Xenopus laevis oocytes " JOURNAL OF ENDOCRINOLOGICAL INVESTIGATION, SUPPLEMENT NO. 4, vol. 21, June 1998, page 77 XP002092888
- HAGENBUCH, B., ET AL. : "functional expression cloning and characterization of the hepatocte Na+/bile acid cotransport system" PROCEEDINGS OF THE NATIONAL ACADEMY OF SCIENCES OF THE USA, vol. 88, December 1991, pages 10629-10633, XP002092889
- HAGENBUCH, B. AND MEIER, P.J.: "molecular cloning, chromosomal localisation, and fuctional charcterization of a human liver Na+/bile acid cotransporter" THE JOURNAL OF CLINICAL INVESTIGATION, vol. 93, no. 3, March 1994, pages 1326-1331, XP002092890
- JACQUEMIN,E., ET AL.: "expression cloning of a rat liver Na+-independent organic anion transporter" PROCEEDINGS OF THE NATIONAL ACADEMY OF SCIENCES OF THE USA, vol. 91, January 1994, pages 133-137, XP002092891
- KULLAK-UBLICK, G.A., ET AL.: "molecular and functional characterization of an organic anion transporting polypeptide cloned from human liver" GASTROENTEROLOGY, vol. 109, 1995, pages 1274-1282, XP002092892
- MEIER,P.J., ET AL. : "substrate specificity of sinusoidal bile acid and organic anion uptake systems in rat and human liver" HEPATOLOGY, vol. 26, no. 6, December 1997, pages 1667-1677, XP002092893

## Description

The invention relates to the field of endocrinology, more specifically to the field of regulating thyroid hormone bioavailability and activity.

Thyroid hormone is essential for the development and homeostasis of different organs, particularly for the regulation of energy metabolism. The follicular cells of the thyroid gland produce predominantly the prohormone thyroxine (3,3',5,5'-tetraiodothyronine, T4) which has little or no biological activity. T4 is activated by enzymatic outer ring deiodination to 3,3',5-triiodothyronine (T3), which is the most if not only bioactive form of thyroid hormone (1-4). Both T4 and T3 are inactivated by enzymatic inner ring deiodination to the metabolites 3,3',5'-triiodothyronine (reverse T3, rT3) and 3,3'-diiodothyronine (3,3'-T2), respectively (1-4).

The three deiodinases involved in these processes show different catalytic profiles, tissue distributions and regulatory functions. They have recently been identified as homologous transmembrane selenoproteins with their active site exposed to the cytoplasm (1-4).

Additional pathways of thyroid hormone metabolism include sulfation and glucuronidation of the phenolic hydroxyl group by transferases located in the cytoplasm and endoplasmic reticulum of different tissues (2,4).

Thyroid hormone is essential for the development of different tissues, for example the brain, and for the regulation of energy metabolism of a wide range if not all tissues throughout life (5) and regulating its bioavailability or activity in various tissues would enable regulating, influencing or interfering with (metabolic) disease in those tissues.

Most of the actions of thyroid hormone are initiated by binding of T3 to nuclear receptors, which changes the interaction of these receptors with regulatory elements of thyroid hormone-responsive genes and, thus, the expression of these genes (5). Although three T3 receptor (TR) isoforms have been identified (TRα1, TRβ1 and TRβ2) with different tissue distributions, their ligand specificities are very similar, which does not allow tissue specific regulation. Moreover, TR antagonists have not been identified-with the possible exception of the anti-arrhythmic drug amiodarone. Consequently, TRs do not appear to be useful targets for the development of (tissue specific) thyroid hormone agonists or antagonists.

However, considering the central role that is played by thyroid hormone in the basic metabolism of a wide variety of cells in a wide range of tissues, use of drugs comprising thyroid hormone agonists and/or antagonists will have benificial effects in many diseases.

Consequently, a need exists to be able to test drugs in bioassays capable of measuring the effect on bioavailability and acitivity of thyroid hormones. For instance, liver or adipose tissue-targeted agonists would be potentially important drugs for the treatment of obesity, myocard-targeted agonists would be very useful for the treatment of heart failure and heart-targeted antagonists would possess important anti-arrhythmic activity and would give acute relieve of thyrotoxic effects on the heart.

The invention provides an isolated and/or recombinant nucleic acid or functional fragment, homologue, orthologue or derivative thereof, corresponding to a gene encoding a thyroid hormone plasma membrane transporting polypeptide. In a preferred embodiment, said polypeptide is at least capable of transporting 3,3',5-triiodothyronine (T3) which is the active hormone transported to and required by most tissues, however, T4 transport is of importance in for example brain tissue. Access of plasma thyroid hormone to intracellular receptors and enzymes requires transport across the cell membrane. On the basis of the lipophilic nature of iodothyronines, it was assumed for a long time that they cross the cell membrane by simple diffusion. However, this ignored the highly polar nature of the alanine side chain which is a formidable obstacle for membrane passage of iodothyronines. During the last two decades, although none were found, overwhelming evidence has accumulated indicating the involvement of plasma membrane transporters in tissue uptake of thyroid hormone (4,6). These have now been provided by the present invention.

Studies using isolated rat hepatocytes have suggested earlier the involvement of separate transporters for T4 and rT3 uptake and for T3 uptake in liver cells (4,6). Uptake of the different iodothyronines has been shown to be energy (ATP)-dependent, but modest decreases in cellular ATP levels lower T4 and rT3 uptake to a larger extent than T3 uptake. Preincubation of hepatocytes with the Na/K-ATPase inhibitor ouabain results in a strong inhibition of the uptake of thyroid hormones, suggesting that this may be a Na⁺-dependent process. However, direct effects of the extracellular Na⁺ concentration on iodothyronine uptake by liver cells have not been demonstrated.

Where in this application the definition "nucleic acid" is used, both RNA and DNA, in single or doublestranded fashion, and nucleic acid hybridising thereto is meant. "Homologue" herein meaning a related nucleic acid that can be found with the same species, "orthologue" herein meaning a related nucleic acid that can be found with another species. "Derivative" herein meaning a nucleic acid that has been derived by genetic modifications, such as deletions, insertions, and mutations from a distinct nucleic acid or fragment thereof. "Corresponding" herein meaning having a nucleic acid sequence homology of at least 30%, more preferably of at least 50 or even 90%. "Functional fragment", when relating to nucleic acid, herein meaning a nucleic acid or part thereof that is functionally or structurally related to or hybridising with a distinct nucleic acid or fragment thereof.

In a preferred embodiment the invention provides an isolated and/or recombinant nucleic acid or functional fragment, homologue, orthologue or derivative thereof, corresponding to a gene encoding a thyroid hormone plasma membrane transporting polypeptide, for example capable of transporting 3,3',5-triiodothyronine, wherein said polypeptide is related to an organic anion transporting peptide such as sodium-independent organic anion-transporting peptide.

The invention furthermore provides a protein encoded by a nucleic acid provided by the invention capable of transporting a thyroid hormone through a plasma membrane.

An example is rat oatp1 which is a 670-aminoacid protein with 12 transmembrane domains and 2 glycosylation sites with an apparent molecular mass of 80 kDa (14-16). Oatp1 is not only expressed in liver but also in kidney and brain. Oatp1 is localized to the basolateral liver cell membrane. Oatp1 is a multispecific transporter mediating the uptake of a wide variety of amphipathic ligands (15,16,25-28), including conjugated and unconjugated bile acids, conjugated steroids (*e.g.* estrone sulfate, estradiol 17β-glucuronide and DHEA sulfate) and other organic anions (*e.g.* the prototypic bromosulfophthalein), but also neutral steroids (*e.g.* aldosterone and cortisol), cardiac glycosides (*e.g.* ouabain) and even organic cations (*e.g.* ajmalinium). Apparent Km values of taurocholate and bromosulfophthalein for rat oatp1 amount to 50 and 1.5 mM, respectively (16).

Transport through oatp1 is not coupled to Na⁺. There is evidence suggesting that oatp1 mediates exchange of intra- and extracellular anions (29). Albumin has been reported to stimulate uptake of ligands through oatp1 but this is controversial (16). Human OATP has recently also been characterized (30) as well as 2 strongly homologous transporters expressed in rat liver, kidney and brain (oatp2; Ref. 31) or exclusively in kidney (OAT-K1; Ref. 32). Less homologous proteins have recently been identified as prostaglandin transporters in rat and human liver (33,34).

The invention furthermore provides a recombinant or synthetic protein or functional fragment thereof encoded by a nucleic acid according to the invention capable of transporting a thyroid hormone. Such a functional fragment for example comprises a thyroid hormone binding site or at least a part of such a site, which is often comprising one or more peptides within the large polypeptide. Expressing recombinant protein and isolating and purifying the resulting protein or fragments thereof is a skill available in the art. For example, the invention provides a cell comprising a recombinant nucleic acid, or fragment thereof, encoding a plasma membrane thyroid hormone transporter, said cell being capable of expressing a protein according to the invention. In a preferred embodiment of the invention. such a cell comprising a recombinant nucleic acid, or fragment thereof, encoding a plasma membrane thyroid hormone transporter, comprises for example a non-human stem cell, allowing generating of a non-human transgenic animal, preferable an experimental animal, preferably a mouse or rat, having been provided with a genome comprising a modified nucleic encoding a (tissue specific) plasma membrane thyroid hormone transporting polypeptide. Generating non-human transgenic animals is in itself a skill known in the art.

The invention also provides a peptide derived from a nucleic acid molecule encoding a plasma membrane thyroid hormone transporter as provided by the invention, In a preferred embodiment, said peptide is a synthetic peptide derived from the sequence of said nucleic acid. Preparing synthetic peptides in general is a skill available in the art. Furthermore, mapping active sites of peptides in general is a skill known in the art, for example, one can use Pepscan techniques or replacement mapping techniques, allowing identification of active sites. It is well known that such peptides can mimic linear or conformational configurations of active sites such as binding sites, and not necessarily need to be derived from a linear sequence alone. In particular, the invention provides a peptide at least comprising (for example mimicking) a thyroid hormone binding site or parts thereof, which can easily be determined now that various thyroid hormone tranporter polypeptides and nucleic acid encoding these are provided.

The identification of transporters involved in the uptake of thyroid hormone in different tissues is not only important in the study of the regulation of these processes in health and disease, but it also provides for the development of tissue-specific thyroid hormone agonists and antagonists which are beneficial in the treatment of conditions such as obesity and cardiovascular diseases. In summary, the invention provides a wide range of tissue-specific thyroid hormone transporters, for example specific for liver, kidney, brain, and other tissues wherein homologues of oatp function as plasma membrane transporter. Ligands or blockers of these various tissue specific thyroid hormone transporters are important candidate drugs for the development of pharmaceutical compounds acting as (tissue specific) agonist or antagonist of thyroid hormone activity, which are herewith provided.

In a preferred embodiment, such ligands or blockers comprise a peptide derived from a nucleic acid or fragment thereof encoding a thyroid hormone transporter as provided by the invention. To derive peptides which act as ligand (agonist) or blocker (antagonist) is a skill known in the art, for example, one can use Pepscan techniques or replacement mapping techniques as well as for example phage-display techniques and screeing of combinatorial libraries, allowing identification of active sites in a polypeptide sequence. In particular, the invention provides a peptide at least comprising an active site capable of binding to or influencing or interfering with the binding and/or transporting of a ligand (such as an amphipathic ligand, neutral steroid, organic anion or even organic cation, preferably of a thyroid hormone nature or functionally equivalent thereto) of a thyroid hormone binding site or part thereof, of a thyroid hormone plasma membrane transporting polypeptide as provided by the invention.

Furthermore, the invention provides a (synthetic) antibody or other binding molecule specifically directed against a plasma membrane thyroid hormone transporting polypeptide as provided by the invention, or at least binding to or interfering with the binding of a ligand of a thyroid hormone binding site or parts thereof, of a thyroid hormone plasma membrane transporting polypeptide as provided by the invention. Generating antibodies or other binding molecules is a skill available in the art, and can be done with classical immunological techniques as well as for example with phage-display techniques.

The invention provides a bioassay or method to identify such candidate drug agonists or antagonists, for example for use in a pharmaceutical compound for treating obesity, heart failure or (tissue specific) hypo- or hyper-thyroidism. Candidate drugs, often first selected or generated via combinatorial chemistry or comprising a peptide as provided by the invention, can now be tested and identified using a method provided by the invention. Such a candidate drug or compound can for example be tested on and selected for its effect on T3 uptake in hepatocytes, or other cells, depending on the specificity of the transporter desired. For use in brain cells, where T3 is autonomously produced, a candidate drug or compound can for example be tested on and selected for its effect on T4 uptake as a precursor for T3. As for example can be seen in several of the figures in the description, the invention provides methods and means to measure thyroid hormone cellular uptake and regulation thereof.

The invention provides use of a nucleic acid according to the invention or of protein, antibody peptides, cells, or animals derived thereof in a method for detecting a pharmaceutical compound for treating a thyroid hormone related disorder or other diseases. In a preferred embodiment, the invention provides a bioassay employing means and methods herein provided, such as a nucleic acid or fragment thereof encoding a plasma membrane thyroid hormone polypeptide or the related protein or (synthetic) peptide, or antibody directed against such a (poly)peptide or its active site or parts thereof, or cells or non-human transgenic animals comprising such nucleic acid or recombinant proteins or peptides, or related synthetic peptide derived from a nucleic acid or fragment thereof encoding a plasma membrane thyroid hormone polypeptide.

A pharmaceutical compound, as provided by the invention, finds its use in a method for treating a wide range of disorders, such as disorders of thyroid metabolism, for example related to brain disorders seen with psychiatric disease, to restore or help develop tissue metabolism or development in premature children, to help restore thyroid hormone function in patients wherein the thyroid has been removed or is dysfunctioning, for example due to a malignancy, to help alleviate non-thyroidal illness, to treat obesity or cardiovascular illness, to name a few. In fact, all disorders or disease wherein the (basic) metabolism of a cell or tissue is affected can be treated with a pharmaceutical compound as provided by the invention, whereby an agonist mainly acts in up regulating metabolism and an antagonist mainly acts in down regulating metabolism. In a preferred embodiment, such a pharmaceutical compound comprises a peptide, preferably a synthetic peptide as provided by the invention, or an antibody or other binding molecule or thyroid hormone analog as provided by the invention capable of binding to or influencing or interfering with the binding or transporting of a ligand of a thyroid hormone plasma membrane transporting polypeptide. In a preferred embodiment, such a compound can bind to or influence or interfere with the binding or transporting of a ligand (for example T3) of a polypeptide which is capable of transporting 3,3',5-triiodothyronine. Examples of such a polypeptide are wherein said polypeptide is related to an organic anion transporting peptide such as sodium-independent organic anion-transporting peptide. Optionally a pharmaceutical compound as provided by the invention has been provided with a carrier or diluent known in the art.

Pharmaceutical compounds comprising tissue specific thyroid hormone agonists/antagonists as provided by the invention may for example be used in treating obesity, heart failure or (tissue specific) hypo- or hyper-thyroidism. Another example is when tissue specific malignancies such as tumours require up- or down-regulation. This requires determining which thyroid hormone transporter is used by the cells in the tissue or malignancy, (for example by biopsy and histochemistry using immunological detection or detection of mRNA expression) and then treating the patient with an agonist/antagonist as provided by the invention which specifically acts through the then determined transporter.

Furthermore, pharmaceutical compounds comprising tissue specific thyroid hormone agonists/antagonists as provided by the invention may be used for the production of a medicament for treating a disorder of thyroid metabolism, non-thyroidal illness, obesity or cardiovascular illness.
The invention also provides use of a nucleic acid or a protein according to the invention for the production of a medicament for treating a thyroid hormone related disorder such as a disorder of thyroid metabolism, non-thyroidal illness, obesity or cardiovascular illness, or other illnesses, for example such medicament is suitable for gene therapy wherein tissue specific thyroid hormone transporter gene or proteins are targeted by gene delivery vehicles to diseased cells or tissues.

The invention is further explained in the drawings and the experimental part without limiting the invention.

### Figure legends

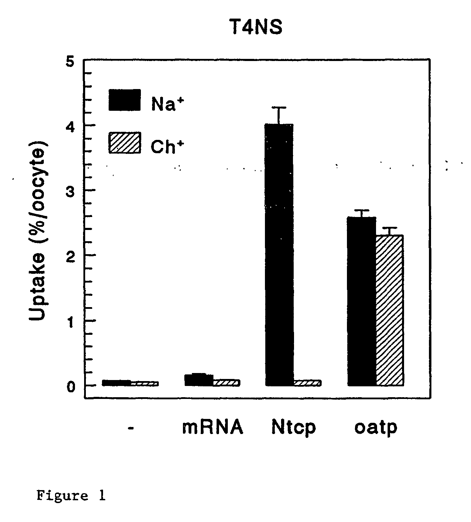

- Fig. 1.: Uptake of T3NS by oocytes injected with 0-2.3 ng Ntcp or oatp1 cRNA. Two days after injection, oocytes were incubated for 1 h at 25 C with 0.1 mM [¹²⁵I]T3NS in Na⁺ or Ch⁺ medium. Data are presented as means ± SEM of 7-10 oocytes.

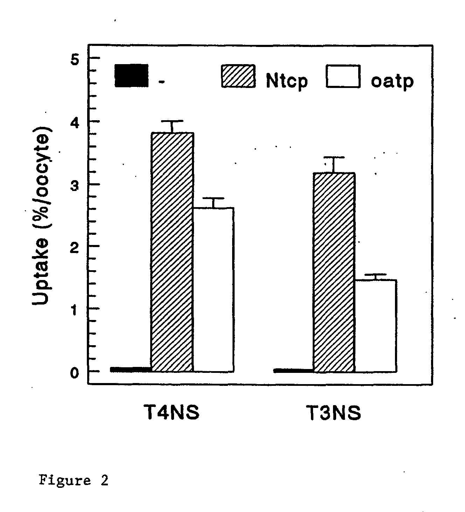

- Fig. 2.: Uptake of T4NS by uninjected oocytes and oocytes injected with 23 ng rat liver mRNA or 2.3 ng Ntcp or oatp1 cRNA. Three days after injection, oocytes were incubated for 1 h at 25 C with 14 nM [¹²⁵I]T4NS in Na⁺ or Ch⁺ medium. Data are presented as means ± SEM of 18-20 oocytes. *) p<0.001 *vs.* uninjected; #) p<0.001 *vs.* Na⁺.

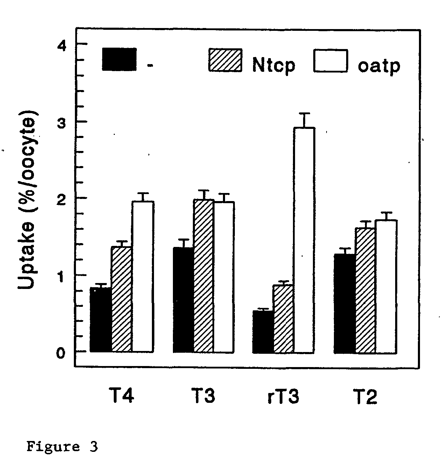

- Fig. 3.: Uptake of iodothyronines by uninjected oocytes and oocytes injected with 2.3 ng Ntcp or oatp1 cRNA. Two days after injection, oocytes were incubated for 1 h at 25 C with 0.1 mM ¹²⁵I-labeled T4, T3, rT3 or 3,3'-T2 in Na⁺ medium. Data are presented as means ± SEM of 16-38 oocytes. *) p<0.005 *vs.* uninjected; **) p<0.001 *vs.* uninjected.

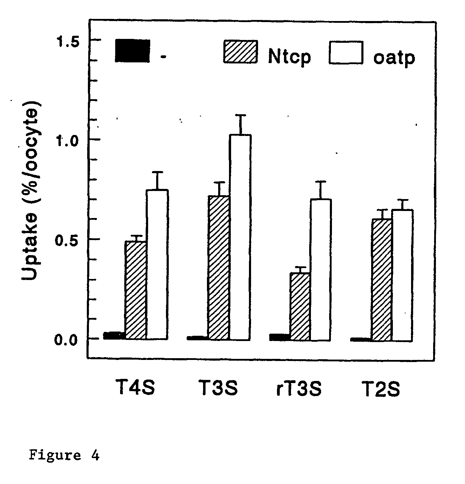

- Fig. 4.: Uptake of iodothyronine sulfates by uninjected oocytes and oocytes injected with 2.3 ng Ntcp or oatp1 cRNA. Two days after injection, oocytes were incubated for 1 h at 25 with 1-2 nM ¹²⁵I-labeled T4S, T3S, rT3S or 3,3'-T2S in Na⁺ medium. Data are presented as means ± SEM of 7-8 oocytes. *) p<0.001 *vs*. uninjected.

### Experimental part.

Thyroid hormone action and metabolism are intracellular events that require transport of the hormone across the plasma membrane. Previous studies in rat hepatocytes have suggested multiple transporters for uptake of the prohormone T4, the active hormone T3 and the inactive metabolites rT3 and 3,3'-T2. In this study we tested possible transport of these iodothyronines and their sulfate (S) conjugates by the Na⁺/taurocholate cotransporting polypeptide (Ntcp) and the Na⁺-independent organic anion transporting polypeptide (oatp1). *Xenopus laevis* oocytes were injected with 2.3 ng Ntcp or oatp1 cRNA, and after 2-3 days they were incubated for 1 h at 25 C with usually 0.1 mM [¹²⁵I]ligand. Ntcp and oatp1 cRNA increased T4S uptake 16- and 25-fold, T3S uptake 53- and 76-fold, rT3S uptake 12- and 26-fold, and 3,3'-T2S uptake 49- and 53-fold, respectively. Ntcp and oatp1 cRNA induced similar increases in uptake of nonsulfated iodothyronines, but the latter showed much higher uptake by uninjected oocytes than the sulfates. The fold increase induced by Ntcp and oatp1 cRNA amounted to 1.9 and 2.8 for T4, 1.7 and 1.7 for T3, 1.8 and 6.0 for rT3, and 1.3 and 1.4 for 3,3'-T2, respectively. Both Ntcp and oatp1 cRNA induced an even greater stimulation of the uptake of iodothyronine sulfamates. The Ntcp cRNA-induced uptake of the iodothyronine derivatives was completely inhibited by replacement of Na⁺ in the medium with choline, whereas the oatp1 cRNA-induced uptake was not affected. These results show that hepatic uptake of thyroid hormones and their metabolites is mediated, at least in part, by Ntcp and oatp1.

In recent years, we have explored the possibility to clone iodothyronine transporters from rat liver using the *Xenopus laevis* oocytes expression system (9-12). We observed modest increases in T4 and T3 uptake by oocytes 3-4 days after injection with rat liver mRNA, in particular the 0.8-2.1 kb size fraction (9). The relatively small stimulation induced by mRNA injection is due to high endogenous uptake of iodothyronines by native oocytes (9). A much lower background uptake was observed with T3 sulfate (T3S), resulting in a larger relative increase after injection of liver mRNA (9). We also tested the non-naturally occuring sulfamate derivatives of T4 (T4NS) and T3 (T3NS) as potentially useful water-soluble ligands for the thyroid hormone transporters. Uptake of T4NS and T3NS was found to be negligible in native oocytes and strongly stimulated after injection of rat liver mRNA (11,12). This uptake was competitively inhibited by relatively high concentrations of T4 and T3, suggesting the involvement of common transporters (11,12). T4NS and T3NS are organic anions, and different multispecific transporters for such compounds have been cloned from rat liver, including the Na⁺/taurocholate cotransporting polypeptide (Ntcp) and the Na⁺-indepedent organic anion transporting polypeptide (oatp1) (13-16). This study was therefore conducted to test the possible involvement of Ntcp and oatp1 in liver uptake of iodothyronine derivatives.

### Materials

T4 and T3 were obtained from Sigma Chemical Co. (St. Louis, MO, USA). rT3 and 3,3'-T2 were obtained from Henning Berlin GmbH (Berlin, Germany). [¹²⁵I]T4 and [¹²⁵I]T3 were purchased from Amersham (Amersham, UK). [¹²⁵I]rT3, [¹²⁵I]3,3'-T2, [¹²⁵I]T4NS, [¹²⁵I]T3NS, [¹²⁵I]T4S, [¹²⁵I]T3S, [¹²⁵I]rT3S and [¹²⁵I]3,3'-T2S were prepared as previously described (17). [¹²⁵I]T4 and [¹²⁵I]rT3 as well as their derivatives were purified on Sephadex LH-20 (Pharmacia, Uppsala, Sweden) immediately before use (17). All other chemicals were of reagent grade.

### Animals

Two-yr-old adult *Xenopus laevis* females were obtained from the Hubrecht Laboratory (Utrecht, The Netherlands). They were maintained in a water-filled tank with three dark sides at a temperature of 18-22 C. A 12-h light, 12-h dark cycle was maintained to reduce seasonal variations in oocyte quality. Frogs were fed twice a week, and water was changed immediately after feeding.

Livers of male Wistar rats were used for mRNA isolation. Animals had free access to food and water and were kept in a controlled environment (21 C) with a 12-h light, 12-h dark cycle.

### RNA preparation

Rat liver poly(A)⁺ RNA was isolated with a commercial kit (Stratagene, La Jolla, CA) according to the manufacturer's protocol. mRNA was stored in water at -80 C at a concentration of 1 mg/ml.

Capped Ntcp and oatp1 copy RNA (cRNA) were prepared from the cDNA clones (13,14) linearized with *Pvu*I and *Xba*I (Boehringer Mannheim, Mannheim, Germany), respectively, using the Ampliscribe T3 and T7 RNA transcription kits (Epicentre Technologies, Madison, WI), respectively. For capping, the m7G[5']ppp[5']G cap analog was used (Epicentre Technologies). cRNA was stored in water at -80 C at a concentration of 0.1 mg/ml.

### Oocyte isolation and RNA injection

Oocytes were prepared as described previously (9). After isolation, the oocytes were sorted on morphological criteria and defolliculated manually. Healthy-looking stage V-VI oocytes (18) were transferred to six-well tissue culture plates and incubated overnight at 18 C in modified Barth's solution [88 mM NaCl, 1 mM KCl, 0.82 mM MgSO₄, 0.4 mM CaCl₂, 0.33 mM Ca(NO₃)₂, 2.4 mM NaHCO₃, and 10 mM HEPES (pH 7.4), containing 20 IU/ml pencillin and 20 mg/ml streptomycin]. The next day, oocytes were injected with 23 nl water containing 23 ng mRNA, 2.3 ng Ntcp cRNA or 2.3 ng oatp1 cRNA using the Nanoject system (Drummond Scientific, Broomall, PA). Injected and uninjected oocytes were maintained for 2-3 days at 18 C in modified Barth's solution.

### Uptake assay

Uptake assays were performed as described previously (9). Eight to 10 oocytes were incubated for 1 h at 25 C with usually 0.1 mM [¹²⁵I]iodothyronine derivative in 0.1 ml incubation buffer (100 mM NaCl or choline chloride, 2 mM KCl, 1 mM CaCl₂, 1 mM MgCl₂, 10 mM HEPES, and 10 mM Tris, pH 7.5). After 1 h, incubation buffer was removed and the oocytes were washed four times with 2.5 ml ice-cold Na⁺-containing incubation buffer supplemented with 0.1% BSA. Oocytes were transferred to new tubes and counted individually. Similar results were obtained in uninjected and water-injected oocytes. Uptake of [³H]taurocholate by oocytes was studied in parallel, showing similar stimulation of Na⁺-dependent transport after injection of Ntcp cRNA and Na⁺-independent transport after injection of oatp1 cRNA as previously reported (not shown; Refs. 13,14).

### Statistics

Data are presented as the mean ± SEM. Statisical significance was evaluated by Student's *t* test for unpaired observations.

### Results

Figure 1 demonstrates that uptake of T3NS by oocytes is stimulated after injection with increasing amounts of Ntcp or oatp1 cRNA. Uptake of T3NS by uninjected oocytes was negligible. T3NS uptake by Ntcp cRNA-injected oocytes was completely inhibited by replacement of Na⁺ in the medium with choline Ch⁺, whereas T3NS uptake by oatp1 cRNA-injected oocytes was not affected by Na⁺ depletion. At all cRNA doses tested, T3NS uptake was stimulated to a higher degree by Ntcp cRNA than by oatp1 cRNA. Both Ntcp and oatp1-mediated uptake of T3NS reached a maximum after injection of »1.5 ng cRNA per oocyte, and in all further experiment 2.3 ng cRNA was injected per oocyte.

Figure 2 shows the effects of injection of 23 ng total rat liver mRNA or 2.3 ng Ntcp or oatp1 cRNA on the uptake of [¹²⁵I]T4NS by oocytes. T4NS uptake by uninjected oocytes was negligible. Injection of liver mRNA resulted in a significant increase in T4NS uptake, and this increase was markedly reduced by substituting Ch⁺ for Na⁺ in the medium. A dramatic increase in T4NS uptake was observed after injection with Ntcp cRNA or oatp1 cRNA. As with T3NS, the increase induced by Ntcp cRNA was larger than that elicited by oatp1 cRNA. Again, T4NS uptake in Ntcp cRNA-injected oocytes was completely blocked in Na⁺ deplete medium, whereas T4NS uptake in oatp1 cRNA-injected oocytes was similar in Na⁺ and Ch⁺ medium. Direct comparison of T4NS and T3NS uptake showed that Ntcp and oatp1 mediated transport of T4NS 16% and 44% better, respectively, than transport of T3NS.

Figure 3 presents the effects of injection of oocytes with Ntcp or oatp1 cRNA on the uptake of unconjugated iodothyronines. In contrast to the sulfamates, marked uptake of the different iodothyronines was observed with uninjected oocytes which was partially inhibited by replacement of Na⁺ in the medium with Ch⁺ (not shown). This endogenous uptake by oocytes was higher with T3 and 3,3'-T2 than with T4 and rT3. On top of this high background, uptake of the different iodothyronines was significantly stimulated after injection of Ntcp or oatp1 cRNA. The Ntcp cRNA-induced uptake was higher for T4 and T3 than for rT3 and 3,3'-T2, varying between 1.3- and 1.9-fold. Injection of oatp1 cRNA produced by far the largest increase in uptake of rT3 (6.0-fold), followed by T4 (2.8-fold), T3 (1.7-fold) and 3,3'-T2 (1.4-fold). Incubation in Na⁺ deplete medium completely inhibited the Ntcp cRNA-stimulated uptake of the different iodothyronines but did not affect the increase induced by oatp1 cRNA. In Ch⁺ medium, the fold increase in iodothyronine uptake induced by oatp1 cRNA was even greater than in Na⁺ medium, *i.e.* 8.5 for rT3, 3.8 for T4, 1.7 for T3, and 1.8 for 3,3'-T2 (not shown).

Finally, Fig. 4 shows the effects of injection of Ntcp or oatp1 cRNA on the uptake of iodothyronine sulfates by oocytes. Uptake of the conjugates by native oocytes was very low compared with the uptake of the nonsulfated compounds. Injection of both Ntcp cRNA and oatp1 cRNA produced dramatic increases in the uptake of the different sulfoconjugates. In absolute terms, these increase were similar in magnitude as those observed with the nonsulfated iodothyronines (Fig. 3). However, because of the lower background uptake by uninjected oocytes the relative increase induced by Ntcp and oatp1 cRNA was much greater for the sulfates than for the native iodothyronines, *i.e.* 16- and 25-fold for T4S, 53- and 76-fold for T3S, 12- and 26-fold for rT3S, and 49- and 53-fold for 3,3'-T2S, respectively. Except for 3,3'-T2S, therefore, uptake of the sulfates was more strongly stimulated after oatp1 cRNA than after Ntcp cRNA injection. T3S uptake by oatp1 cRNA-injected oocytes was »50% higher than that of the other sulfates. In Ntcp cRNA-injected oocytes uptake decreased in the order T3S » 3,3'-T2S > T4S > rT3S.

### Discussion

Previous studies of the uptake of iodothyronines by rat and human hepatocytes have suggested the involvement of at least 2 active transporters, one mediating the uptake of T4 and rT3 and the other mediating the uptake of T3 (4,6). This was based on observations that uptake of T4 and rT3 was inhibited by relatively small decreases in cellular ATP, whereas inhibition of T3 required larger decreases in ATP content (7). Uptake of all iodothyronines was also found to be inhibited in the presence of 0.5 mM ouabaine, which was thought to reflect the Na⁺ dependence of the transporters (8). The ATP dependence of the transport of the different iodothyronines could then reflect the requirement of ATP to maintain the Na⁺ gradient across the cell membrane. Uptake of the iodothyronines by rat hepatocytes was also shown to be dependent on the presence of albumin in the medium, perhaps by facilitating the diffusion of the lipophilic hormones through the unstirred water layer around the cells (35). Based on the concentrations of non-protein-bound hormone, apparent Km values of the different iodothyronines for the rat liver cell membrane transporters were estimated to be in the nanomolar range (35). Although T3 was a competitive inhibitor of the uptake of T4 and *vice versa,* the Ki and Km values were different for each hormone (35). This is in agreement with the involvement of multiple transporters (4,6).

Our findings demonstrate marked stimulation of the uptake of native iodothyronines as well as their Na-sulfonated (sulfamate) and 4'-OH-sulfonated (sulfate) derivatives after injection of oocytes with cRNA coding for Ntcp or oatp1. The Na⁺ dependence of Ntcp and the Na⁺ independence of oatp1 were confirmed with all these ligands. The largest stimulation was observed with the sulfamates which showed higher transport rates in Ntcp cRNA than in oatp1 cRNA-injected oocytes, with a slight preference of T4NS over T3NS. Maximum uptake was observed for both Ntcp and oatp1 after injection of »1.5 ng cRNA per oocyte. Since the efficiency of the translation of the cRNAs and the processing of the proteins is unknown, differences in uptake rates induced by these messengers cannot be interpreted in terms of molar transport rates.

Although smaller in magnitude, the stimulation of the uptake of the different iodothyronine sulfates in Ntcp or oatp1 cRNA-injected oocytes was as dramatic as that observed with the sulfamates. In general, uptake of the sulfates was induced to a larger extent by oatp1 cRNA than by Ntcp cRNA. T3S showed the highest uptake rates, although differences with the other sulfoconjugates were less than 2-fold. In contrast to the sulfamates, iodothyronine sulfates are naturally occuring thyroid hormone metabolites. Sulfation plays an important role in the metabolism of thyroid hormone (4,36). It facilitates the type I deiodinase-catalyzed inner ring deiodination (degradation) of the prohormone T4 and the active hormone T3, whereas it blocks the outer ring deiodination (activation) of T4 to T3 by that same enzyme (4,36). Our results suggest that both Ntcp and oatp1 contribute importantly to the uptake of T4S and T3S by rat hepatocytes, thus allowing their degradation by the type I deiodinase located in the endoplasmic reticulum (1-4).

A particular finding of our study is the stimulation of the uptake of the different native iodothyronines by injection of Ntcp or oatp1 cRNA. Although the relative increase in uptake of the iodothyronines by either cRNA was less impressive than that observed with the sulfamates and the sulfates, this was primarily due to the much higher endogenous uptake by uninjected oocytes. In fact, the increase in uptake of the different iodothyronines induced by Ntcp or oatp1 cRNA was similar in magnitude as that seen with the sulfates. The degree of stimulation of iodothyronine uptake by oatp1 cRNA was equal to or larger than the increase induced by Ntcp cRNA. By far the largest stimulation occurred after injection of oatp1 cRNA using rT3 as the ligand.

We found that uptake of the different iodothyronine derivatives in Ntcp or oatp1 cRNA-injected oocytes showed no sign of saturation at 0.1 mM ligand concentration (not shown), which is higher than the apparent Km values determined for T4 and T3 uptake in isolated hepatocytes (35). Previous studies have also suggested that uptake of T4 and T3 by rat hepatocytes is largely Na⁺ dependent, based on the major inhibitory effect of the Na/K-ATPase inhibitor ouabain (4,8). However, it is clear that ouabain may also inhibit uptake of iodothyronines directly by competition for oatp-like transporters (25,26,31).

There is much evidence suggesting that plasma membrane transport plays a rate-limiting role in the metabolism and action of thyroid hormone in liver and other tissues (6). In fasting and nonthyroidal illness, peripheral conversion of T4 to T3 is diminished resulting in decreased serum T3 concentrations (37). This is thought to represent a beneficial adaptation mechanism, resulting in decreased energy expenditure and protein breakdown, and, thus, in conservation of tissue function (37). Strong evidence points to reduced uptake of T4 in T3-producing tissues such as the liver as an important mechanism for the decreased T3 production in fasting and nonthyroidal illness (6,37). This may be due to reduced expression of transporters or reduced tissue ATP content, but it may also result from inhibition of hepatic uptake of T4 by circulating factors, such as bilirubin, free fatty acids, 3-carboxy-4-methyl-5-propyl-2-furanpropanoic acid (CMPF) and indoxyl sulfate, the concentrations of which are increased in fasting and/or illness (6,37-39). Hepatic uptake of T4 is also inhibited by pharmaceuticals, such as the antiarrhythmic drug amiodarone and the X-ray contrast agent iopanoic acid, which are known to induce a decrease in serum T3 (6,40-42). It has been postulated that a decrease in cellular reduced glutathione (GSH), which may be a physiological cofactor of the type I deiodinase, could also contribute to the decreased peripheral T3 production during illness and fasting (43). Intriguingly, uptake of organic anions by oatp1 has recently been shown to take place in exchange for intracellular GSH (44).

### REFERENCES

1. Larsen, P.R. and M.J. Berry. 1995. Nutritional and hormonal regulation of thyroid hormone deiodinases. *Annu. Rev. Nutr.* 15: 323-352.
2. Leonard, J.L. and J. Köhrle. 1996. Intracellular pathways of iodothyronine metabolism. *In* The Thyroid, L.E. Braverman and R. Utiger, editors. Lippincott-Raven, Philadelphia, 125-161.
3. St.Germain, D.L. and V.A. Galton. 1997. The deiodinase family of selenoproteins. *Thyroid* 7: 655-688.
4. Hennemann, G. and T.J. Visser. 1997. Thyroid hormone synthesis, plasma membrane transport and metabolism. *In* Handbook of Experimental Pharmacology, Vol 128; Pharmacotherapeutics of the Thyroid Gland, A.P. Weetman and A. Grossman, editors. Springer, Berlin, 75-117.
5. Oppenheimer, J.H., H.L. Schwartz and K.A. Strait. 1996. The molecular basis of thyroid hormone actions. *In* The Thyroid, L.E. Braverman and R. Utiger, editors. Lippincott-Raven, Philadelphia, 162-184.
6. Hennemann, G., M.E. Everts, M. de Jong, C.F. Lim, E.P. Krenning and R. Docter. 1998. The significance of plasma membrane transport in the bioavailablity of thyroid hormone. *Clin. Endocrinol.* 48: 1-8.
7. Krenning, E.P., R. Docter, H.F. Bernard, T.J. Visser and G. Hennemann. 1982. Decreased transport of thyroxine (T4), 3,3',5-triiodothyronine (T3) and 3,3',5'-triiodothyronine (rT3) into rat hepatocytes in primary culture due to a decrease of cellular ATP content and various drugs. *FEBS Lett.* 140: 229-233.
8. Hennemann, G., E.P. Krenning, M. Polhuys, J.A. Mol, B.F. Bernard, T.J. Visser and R. Docter. 1986. Carrier-mediated transport of thyroid hormone into rat hepatocytes is rate-limiting in total cellular uptake and metabolism. *Endocrinology* 119: 1870-1872.
9. Docter, R., E.C.H. Friesema, P.G.J. van Stralen, E.P. Krenning, M.E. Everts, T.J. Visser and G. Hennemann. 1997. Expression of rat liver cell membrane transporters for thyroid hormone in Xenopus laevis oocytes. *Endocrinology* 138: 1841-1846.
10. Friesema, E.C.H., R. Docter, E.P. Krenning, M.E. Everts, G. Hennemann and T.J. Visser. 1998. Rapid sulfation of reverse triiodothyronine in native Xenopus laevis oocytes. *Endocrinology* 139: 596-600.
11. Friesema, E.C.H., E.P.C.M. Moerings, G. Hennemann, T.J. Visser and R. Docter. 1997. Transport of T4 sulfamate (T4NS) and T3 sulfamate (T3NS) into Xenopus laevis oocytes induced by injection of rat liver mRNA. *J. Endocrinol. Invest.* 20 (Suppl): 34.
12. Friesema, E.C.H., E.P.C.M. Moerings, G. Hennemann and R. Docter. 1998. Effects of organic anions (bile acids) on T3 sulfamate (T3NS) and T4 sulfamate (T4NS) uptake into Xenopus laevis oocytes. *J. Endocrinol. Invest.* 21 (Suppl): 77.
13. Hagenbuch, B., B. Stieger, M. Foguet, H. Lübbert and P.J. Meier. 1991. Functional expression cloning and characterization of the hepatocyte Na⁺/bile acid cotransport system. *Proc. Natl. Acad. Sci. USA.* 88: 10629-10633.
14. Jacquemin, E., B. Hagenbuch, B. Stieger, A.W. Wolkoff and P.J. Meier. 1994. Expression cloning of a rat liver Na⁺-independent organic anion transporter. *Proc. Natl. Acad. Sci. USA 91:* 133-137.
15. Hagenbuch, B. 1997. Molecular properties of hepatic uptake systems for bile acids and organic anions. *J. Membrane Biol.* 160: 1-8.
16. Meier, P.J. 1995. Molecular mechanisms of hepatic bile salt transport from sinusoidal blood into bile. *Am. J. Physiol.* 269: G801-G812.
17 Mol, J.A. and T.J. Visser. 1985. Synthesis and some properties of sulfate esters and sulfamates of iodothyronines. *Endocrinology* 117: 1-7.
18 Dumont, J.A. 1972. Oogenesis in *Xenopus laevis* (Daudin) I. Stages of oocyte development in laboratory maintained animals. *J. Morphol.* 136: 153-180.
19. Schroeder, A., U. Eckhardt, B. Stieger, R. Tynes, C.D. Schteingart, A.F. Hofmann, P.J. Meier and B. Hagenbuch. 1998. Substrate specificity of rat liver Na⁺-bile salt cotransporter in enopus laevis oocytes and CHO cells. *Am. J. Physiol.* 274: G370-G375.
20. Shneider, B.L., P.A. Dawson, D.M. Christie, W. Hardikar, M.H. Wong and F.J. Suchy. 1995. Cloning and molecular characterization of the ontogeny of a rat ileal sodium-dependent bile acid transporter. *J. Clin. Invest.* 95: 745-754.
21. Shneider, B.L., K.D.R. Setchell and M.W. Crossman. 1997. Fetal and neonatal expression of the apical sodium-dependent bile acid transporter in the rat ileum and kidney. *Pediat. Res.* 42: 189-194.
22. Hagenbuch, B. and P.J. Meier. 1994. Molecular cloning, chromosomal localization, and functional characterization of a human liver Na⁺/bile acid cotransporter. *J. Clin. Invest.* 93: 1326-1331.
23. Wong, M.H., P. Oelkers and P.A. Dawson. 1995. Identification of a mutation in the ileal sodium-dependent bile acid transporter gene that abolishes transport activity. *J. Biol. Chem.* 270: 27228-27234.
24. Craddock, A.L., M.W. Love, R.W. Daniel, L.C. Kirby, H.C. Walters, M.H. Wong and P.A. Dawson. 1998. Expression and transport properties of the human ileal and renal sodium-dependent bile acid transporter. *Am J Physiol* 274: G157-G169.
25. Bossuyt, X., M. Müller and P.J. Meier. 1996. Multispecific amphipathic substrate transport by an organic anion transporter of human liver. *J. Hepatol.* 25: 733-738.
26. Bossuyt, X., M. Muller, B. Hagenbuch and P.J. Meier. 1996. Polyspecific drug and steroid clearance by an organic anion transporter of mammalian liver. *J. Pharmacol. Exp. Ther.* 276: 891-896.
27. Kanai, N., R. Lu, Y. Bao, A.W. Wolkoff and V.L. Schuster. 1996. Transient expression of oatp anion transporter in mammalian cells: identification of candidate substrates. *Am. J. Physiol.* 270: F319-F325.
28. Kullak-Ublick, G.A., T. Fisch, M. Oswald, B. Hagenbuch, P.J. Meier, U. Beuers and G. Paumgartner. 1998. Dehydroepiandrosterone sulfate (DHEAS): identification of a carrier protein in human liver and brain. *FEBS Lett.* 424: 173-176.
29. Satlin, L.M., V. Amin and A.W. Wolkoff. 1997. Organic anion transporting polypeptide mediates organic anion/HCO₃⁻ exchange. *J. Biol. Chem.* 272: 26340-26345.
30. Kullak-Ublick, G., B. Hagenbuch, B. Stieger, C.D. Schteigart, A.F. Hofmann, A.W. Wolkoff and P.J. Meier. 1995. Molecular and functional characterization of an organic anion transporting polypeptide cloned from human liver. *Gastroenterology* 109: 1274-1282.
31. Noé, B., B. Hagenbuch, B. Stieger and P.J. Meier. 1997. Isolation of a multispecific organic anion and cardiac glycoside transporter from rat brain. *Proc. Natl. Acad. Sci. USA* 94: 10346-10350.
32. Saito, H., S. Masuda and K. Inui. 1996. Cloning and functional characterization of a novel rat organic anion transporter mediating basolateral uptake of methotrexate in the kidney. *J. Biol. Chem.* 271: 20719-20725.
33. Kanai, N., R. Lu, J.A. Satriano, Y. Bao, A.W. Wolkoff and V.L. Schuster. 1995. Identification and characterization of a prostaglandin transporter. *Science* 268: 866-869.
34. Lu, R., N. Kanai, Y. Bao and V.L. Schuster. 1996. Cloning, in vitro expression, and tissue distribution of a human prostaglandin transporter cDNA (hPGT). *J. Clin. Invest.* 98: 1142-1149.
35. Krenning, E.P., R. Docter, H.F. Bernard, T.J. Visser and G. Hennemann G. 1981. Characteristics of active transport of thyroid hormone into rat hepatocytes. *Biochim. Biophys. Acta* 676: 314-320.
36. Visser, T.J. 1994. Role of sulfation in thyroid hormone metabolism. *Chem. Biol. Interact.* 92: 293-303.
37. Docter, R., E.P. Krenning, M. de Jong and G. Hennemann. 1993. The sick euthyroid syndrome: changes in thyroid hormone parameters and hormone metabolism. *Clin. Endocrinol*. 39: 499-518.
38. Lim, C.F., H.F. Bernard, M. de Jong, R. Docter, E.P. Krenning and G. Hennemann. 1993. A furan fatty acid and indoxyl sulfate are the putative inhibitors of thyroxine hepatocyte transport in uremia. *J. Clin. Endocrinol. Metab.* 76: 318-324.
39. Lim, C.F., R. Docter, T.J. Visser, E.P. Krenning, B. Bernard, H. van Toor, M. de Jong and G. Hennemann. 1993. Inhibition of thyroxine transport into cultured rat hepatocytes by serum of nonuremic critically ill patients: effects of bilirubin and nonesterified fatty acids. *J. Clin. Endocrinol. Metab.* 76: 1165-1172.
40. De Jong, M., R. Docter, H. van der Hoek, E. Krenning, D. van der Heide, C. Quero, P. Plaisier, R. Vos and G. Hennemann. 1994. Different effects of amiodarone on transport of T4 and T3 into the perfuse rat liver. *Am. J. Physiol.* 266: E44-E49.
41. Wiersinga, W.M. 1997. Amiodarone and the thyroid. *In* Handbook of Experimental Pharmacology, Vol 128; Pharmacotherapeutics of the Thyroid Gland, A.P. Weetman and A. Grossman, editors. Springer, Berlin, 225-287.
42. Surks, M.I. and R. Sievert. 1995. Drugs and thyroid function. *N. Eng. J. Med.* 333: 1688-1694.
43. Visser, T.J. 1990. The role of glutathione in the enzymatic deiodination of thyroid hormone. *In* Glutathione: Metabolism and Physiological Functions, J. Viña, editor. CRC Press, Boca Raton, 317-333.
44. Li, L., T.K. Lee, P.J. Meier and N. Ballatori. 1998 Identification of glutathione as a driving force and leukotriene C4 as a substrate for oatp1, the hepatic sinusoidal organic solute transporter. *J. Biol. Chem.* 273: 16184-16191.

Abbreviations used herein are: Ch⁺, choline; GSH, reduced glutathione; Ntcp, Na⁺/taurocholate cotransporting polypeptide; oatp, organic anion transporting polypeptide; T4, thyroxine; T4S, T4 sulfate; T4NS, T4 sulfamate; T3, 3,3',5-triiodothyronine; T3S, T3 sulfate; T3NS, T3 sulfamate; rT3 (reverse T3), 3,3',5'-triiodothyronine; rT3S, rT3 sulfate; 3,3'-T2, 3,3'-diiodothyronine; 3,3'-T2S, 3,3'-T2 sulfate.

## Claims

1. In vitro use of a Na+-independent organic anion transporting polypeptide (oatp) or a functional fragment thereof for providing uptake in a cell of a native iodothyronine or a 4'-OH- sulfonated (sulfate) derivative thereof.

2. A use according to claim 1, wherein said native iodothyronine is 3,3',5,5'-tetraiodothyronine (T4) or 3,3',5-triiodothyronine (T3) or 3,3',5'-triiodothyronine (rT3) or 3,3'-diiodothyronine (3,3'-T2).

3. A use according to claim 1 or 2, wherein said cell is provided with an isolated and/or recombinant nucleic acid or functional fragment thereof encoding said oatp.

4. A use according to claim 3, wherein said isolated and/or recombinant nucleic acid or functional fragment thereof is cRNA.

5. A use according to any one of claims 1 to 4, wherein said cell is a non-human oocyte.

6. A bioassay to identify or detect a candidate drug capable of
influencing uptake in a cell of a native iodothyronine or a 4'-OH-sulfonated (sulfate) derivative thereof comprising providing said cell with oatp or a functional fragment thereof and determining whether said candidate drug influences the uptake in said cell of a native iodothyronine or a 4'-OH- sulfonated (sulfate) derivative thereof.

7. A bioassay according to claim 6, wherein said native iodothyronine is T4 or T3 or rT3 or 3,3'-T2.

8. A bioassay according to claim 6 or 7, wherein said cell is provided with an isolated and/or recombinant nucleic acid or functional fragment thereof encoding said oatp.

9. A bioassay according to claim 8, wherein said isolated and/or recombinant nucleic acid or functional fragment thereof is cRNA.

10. A pharmaceutical compound capable of influencing the transporting by oatp of a native iodothyronine or a 4'-OH-sulfonated (sulfate) derivative thereof, wherein said compound is an oatp polypeptide or functional fragment thereof or wherein said compound is an antibody directed against an oatp protein or directed against a functional fragment of said protein.

11. A pharmaceutical compound according to claim 10, wherein said native iodothyronine is 3,3',5,5'-tetraiodothyronine (T4) or 3,3',5-triiodothyronine (T3) or 3,3',5'-triiodothyronine (rT3) or 3,3'-diiodothyronine (3,3'-T2).

12. Use of a pharmaceutical compound according to claim 10 or 11 for the production of a medicament for treating a thyroid hormone related disorder.

13. Use of a pharmaceutical compound according to claim 10 or 11 for the production of a medicament for treating obesity.

14. Use of a pharmaceutical compound according to claim 10 or 11 for the production of a medicament for treating cardiovascular illness.

15. Use of an isolated and/or recombinant nucleic acid or functional fragment thereof encoding oatp for the production of a medicament for treating a thyroid hormone related disorder.

16. Use of an isolated and/or recombinant nucleic acid or functional fragment thereof encoding oatp for the production of a medicament for treating obesity

17. Use of an isolated and/or recombinant nucleic acid or functional fragment thereof encoding oatp for the production of a medicament for treating cardiovascular illness.

18. Use according to any one of claims 12 to 17 wherein said medicament is suitable for gene therapy wherein tissue specific thyroid hormone transporter gene or proteins are targeted by gene delivery vehicles to diseased cells or tissues.

## Patentansprüche

1. In vitro-Verwendung eines Na+-unabhängigen organischen Anionentransportpolypeptids (oatp) oder eines funktionellen Fragments davon zum Bereitstellen der Aufnahme eines nativen Jodthyronins oder eines 4'-OH-sulfonierten (Sulfat) Derivats davon in eine Zelle.

2. Verwendung gemäß Anspruch 1, wobei das native Jodthyronin 3,3',5,5'-Tetrajodthyronin (T4) oder 3,3',5-Trijodthyronin (T3) oder 3,3',5'-Trijodthyronin (rT3) oder 3,3'-Dijodthyronin (3,3'-T2) ist.

3. Verwendung gemäß Anspruch 1 oder 2, wobei die Zelle mit einer isolierten und/oder rekombinanten Nukleinsäure oder einem funktionellen Fragment davon, die/das das oatp kodiert, versehen ist.

4. Verwendung gemäß Anspruch 3, wobei die isolierte und/oder rekombinante Nukleinsäure oder das funktionelle Fragment davon cRNA ist.

5. Verwendung gemäß einem der Ansprüche 1 bis 4, wobei die Zelle eine nichtmenschliche Oozyte ist.

6. Bioassay zum Identifizieren oder Detektieren eines Arzneimittelkandidaten, der die Fähigkeit besitzt, die Aufnahme eines nativen Jodthyronins oder eines 4'-OH-sulfonierten (Sulfat) Derivats davon in eine Zelle zu beeinflussen, umfassend Bereitstellen der Zelle mit oatp oder einem funktionellen Fragment davon und Bestimmen, ob der Arzneimittelkandidat die Aufnahme eines nativen Jodthyronins oder eines 4'-OH-sulfonierten (Sulfat) Derivats davon in die Zelle beeinflusst.

7. Bioassay gemäß Anspruch 6, wobei das native Jodthyronin T4 oder T3 oder rT3 oder 3,3'-T2 ist.

8. Bioassay gemäß Anspruch 6 oder 7, wobei die Zelle mit einer isolierten und/oder rekombinanten Nukleinsäure oder einem funktionellen Fragment davon, die/das das oatp kodiert, versehen ist.

9. Bioassay gemäß Anspruch 8, wobei die isolierte und/oder rekombinante Nukleinsäure oder das funktionelle Fragment davon cRNA ist.

10. Pharmazeutische Verbindung, welche die Fähigkeit besitzt, das Transportieren durch oatp eines nativen Jodthyronins oder eines 4'-OH-sulfonierten (Sulfat) Derivats davon zu beeinflussen, wobei die Verbindung ein oatp Polypeptid oder ein funktionelles Fragment davon ist oder wobei die Verbindung ein Antikörper ist, der gegen ein oatp Protein oder gegen ein funktionelles Fragment des Proteins gerichtet ist.

11. Pharmazeutische Verbindung gemäß Anspruch 10, wobei das native Jodthyronin 3,3',5,5'-Tetrajodthyronin (T4) oder 3,3',5-Trijodthyronin (T3) oder 3,3',5'-Trijodthyronin (rT3) oder 3,3'-Dijodthyronin (3,3'-T2) ist.

12. Verwendung einer pharmazeutischen Verbindung gemäß Anspruch 10 oder 11 für die Herstellung eines Medikaments zur Behandlung einer Störung, die mit einem Thyroidhormon in Verbindung steht.

13. Verwendung einer pharmazeutischen Verbindung gemäß Anspruch 10 oder 11 für die Herstellung eines Medikaments zur Behandlung von Fettleibigkeit.

14. Verwendung einer pharmazeutischen Verbindung gemäß Anspruch 10 oder 11 für die Herstellung eines Medikaments zur Behandlung von Herzkreislauferkrankung.

15. Verwendung einer isolierten und/oder rekombinanten Nukleinsäure oder eines funktionellen Fragments davon, die/das das oatp kodiert, für die Herstellung eines Medikaments zur Behandlung einer Störung, die mit einem Thyroidhormon in Verbindung steht.

16. Verwendung einer isolierten und/oder rekombinanten Nukleinsäure oder eines funktionellen Fragments davon, die/das oatp kodiert, für die Herstellung eines Medikaments zur Behandlung von Fettleibigkeit.

17. Verwendung einer isolierten und/oder rekombinanten Nukleinsäure oder eines funktionellen Fragments davon, die/das oatp kodiert, für die Herstellung eines Medikaments zur Behandlung von Herzkreislauferkrankung.

18. Verwendung gemäß einem der Ansprüche 12 bis 17, wobei das Medikament für die Gentherapie geeignet ist, wobei ein gewebespezifisches Thyroidhormontransportergen oder -proteine durch Genverabreichungsvehikel auf erkrankte Zellen oder Gewebe abzielen.

## Revendications

1. Utilisation in vitro d'un polypeptide transportant des anions organiques indépendant de Na⁺ (oatp) ou d'un fragment fonctionnel de celui-ci pour permettre le captage dans une cellule d'une iodothyronine naturelle ou d'un dérivé 4'-OH-sulfoné (sulfate) de celle-ci.

2. Utilisation selon la revendication 1, dans laquelle ladite iodothyronine naturelle est la 3,3',5,5'-tétraiodothyronine (T4) ou la 3,3',5-triiodothyronine (T3) ou la 3,3',5'-triiodothyronine (rT3) ou la 3,3'-diiodothyronine (3,3'-T2).

3. Utilisation selon la revendication 1 ou 2, dans laquelle ladite cellule est munie d'un acide nucléique isolé et/ou recombinant ou d'un fragment fonctionnel de celui-ci codant ledit oatp.

4. Utilisation selon la revendication 3, dans laquelle ledit acide nucléique isolé et/ou recombinant ou un fragment fonctionnel de celui-ci est l'ARNc.

5. Utilisation selon une quelconque des revendications 1 à 4, dans laquelle ladite cellule est un ovocyte non humain.

6. Essai biologique pour identifier ou détecter un médicament candidat capable d'influencer le captage dans une cellule d'une iodothyronine naturelle ou d'un dérivé 4'-OH-sulfoné (sulfate) de celle-ci consistant à fournir ladite cellule avec un oatp ou un fragment fonctionnel de celui-ci et à déterminer si ledit médicament candidat influence le captage dans ladite cellule d'une iodothyronine naturelle ou d'un dérivé 4'-OH-sulfoné (sulfate) de celle-ci.

7. Essai biologique selon la revendication 6, dans lequel ladite iodothyronine naturelle est T4 ou T3 ou rT3 ou 3,3'-T2.

8. Essai biologique selon la revendication 6 ou 7, dans lequel ladite cellule est munie d'un acide nucléique isolé et/ou recombinant ou d'un fragment fonctionnel de celui-ci codant ledit oatp.

9. Essai biologique selon la revendication 8, dans lequel ledit acide nucléique isolé et/ou recombinant ou un fragment fonctionnel de celui-ci est l'ARNc.

10. Composé pharmaceutique capable d'influencer le transport par oatp d'une iodothyronine naturelle ou d'un dérivé 4'-OH-sulfoné (sulfate) de celle-ci, dans lequel ledit composé est un polypeptide oatp ou un fragment fonctionnel de celui-ci ou dans lequel ledit composé est un anticorps dirigé contre une protéine oatp ou dirigé contre un fragment fonctionnel de ladite protéine.

11. Composé pharmaceutique selon la revendication 10, dans lequel ladite iodothyronine naturelle est la 3,3',5,5'-tétraiodothyronine (T4) ou la 3,3',5-trüodothyronine (T3) ou la 3,3',5'-triiodothyronine (rT3) ou la 3,3'-diiodothyronine (3,3'-T2).

12. Utilisation d'un composé pharmaceutique selon la revendication 10 ou 11 pour la production d'un médicament pour traiter un trouble concernant l'hormone thyroïdienne.

13. Utilisation d'un composé pharmaceutique selon la revendication 10 ou 11 pour la production d'un médicament pour traiter l'obésité.

14. Utilisation d'un composé pharmaceutique selon la revendication 10 ou 11 pour la production d'un médicament pour traiter des maladies cardiovasculaires.

15. Utilisation d'un acide nucléique isolé et/ou recombinant ou d'un fragment fonctionnel de celui-ci codant oatp pour la production d'un médicament pour traiter un trouble relatif à l'hormone thyroïdienne.

16. Utilisation d'un acide nucléique isolé et/ou recombinant ou d'un fragment fonctionnel de celui-ci codant oatp pour la production d'un médicament pour le traitement de l'obésité.

17. Utilisation d'un acide nucléique isolé et/ou recombinant ou d'un fragment fonctionnel de celui-ci codant oatp pour la production d'un médicament pour le traitement de maladies cardiovasculaires.

18. Utilisation selon une quelconque des revendications 12 à 17, dans laquelle ledit médicament est approprié pour la thérapie génique où des protéines ou gènes transporteurs de l'hormone thyroïdienne spécifique de tissu sont ciblés par des véhicules de délivrance de gènes à des cellules ou tissus affectés.
